# EUROPEAN PATENT APPLICATION

(11) **EP 3 549 626 A1**
(43) Date of publication of application: **09.10.2019**
(21) Application number: 17861206.5
(22) Date of filing: 17.07.2017
(51) Int. Cl.: A61M 5/315, A61M 5/178

(54) **PHARMACEUTICAL PRODUCT DELIVERY DEVICE AND ANTI-RETURN MECHANISM THEREOF**

(30) Priority: 05.12.2016 CN 201621324671 U
(71) Applicant: Gansu Changee Bio-pharmaceutical Limited Company, Tianshui, Gansu 741020 (CN)
(72) Inventor: ZHU, Xiaopeng, Tianshui Gansu 741020 (CN); HAN, Yitao, Tianshui Gansu 741020 (CN); PAN, Junfeng, Tianshui Gansu 741020 (CN); LIU, Jian, Tianshui Gansu 741020 (CN)
(74) Representative: Schnappauf, Georg
(86) International application number: PCT/CN2017/093103
(87) International publication number: WO 2018/103338

(57) **Abstract**

An anti-back mechanism for a drug delivery device according to the present application includes a retaining groove (7), a position retaining protrusion (6) and a clamping groove. The retaining groove (7) is provided on a push rod (4). The position retaining protrusion (6) is provided in the drug delivery device. The clamping groove has an accommodation cavity into which the position retaining protrusion (6) is inserted. The position retaining protrusion (6) is provided on a ring having a notch and may be clamped with respect to the push rod (4) when the position retaining protrusion (6) is squeezed. When the position retaining protrusion (6) is subjected to a reverse thrust from a cartridge holder (1) of the drug delivery device, the position retaining protrusion (6) may move in a direction opposite to an injection direction, be inserted into the clamping groove to be clamped, and be hooked to the retaining groove (7) to be locked. For the anti-back mechanism, since the cartridge holder of the drug delivery device is squeezed, the position of the position retaining protrusion (6) is changed, the position retaining protrusion (6) is pushed into the clamping groove and clamped to the retaining groove (7), finally, the position retaining protrusion (6) is hooked to the retaining groove (7) to be locked, thereby achieving the effects of fastening, anti-reversing and clamping. Hereby, a phenomenon of reversing of the push rod (4) is prevented from occurring.

## Description

### FIELD

The present application claims the benefit of priority to Chinese patent application No. 201621324671.4 titled "DRUG DELIVERY DEVICE AND ANTI-BACK DEVICE THEREFOR", filed with the Chinese State Intellectual Property Office on December 5, 2016, the entire disclosure of which is incorporated herein by reference.

### BACKGROUND

In the market, there are many drug delivery devices, for example, injectors, cartridges and other containers. The main structure of a drug delivery device includes a core, a push rod and a liner. The drug is arranged in the liner, and the push rod is pushed so as to force and inject the drug into a human body. In operation, the push rod is pushed by an external force to squeeze a rubber bung of the core in the device, and the push rod advances to push liquid inside the rubber bung out. According to the principle of a reverse thrust, when the external force provided on the push rod is stopped, a certain reverse thrust may be generated by liquid inside the core and the rubber bung in the device. If the external force is no longer provided after an injection process is ended, the push rod is apt to reverse. For a drug delivery device with a measurement function, a great error may present due to measurement by the push rod. In addition, reversing of the push rod may further cause air to enter an interior of the device, thereby causing adverse influences.

Therefore, an issue to be addressed presently by the person skilled in the art is to provide a drug delivery device and an anti-back mechanism thereof, which allow the push rod to remain at its original position after use.

### SUMMARY

A drug delivery device and an anti-back mechanism thereof are provided according to the present application. The anti-back mechanism can allow a push rod to remain at its original position after use. The drug delivery device employing the anti-back mechanism can prevent the push rod from reversing.

In order to address the above technical issue, an anti-back mechanism for a drug delivery device is provided according to the present application. The anti-back mechanism includes a retaining groove, a position retaining protrusion and a clamping groove. The retaining groove is provided on a push rod. The position retaining protrusion is arranged in the drug delivery device. The clamping groove has an accommodation cavity into which the position retaining protrusion is inserted. The clamping groove is arranged at an end, close to a rubber bung, of the push rod. The position retaining protrusion is arranged on a ring having a notch, and may be clamped with respect to the push rod when the position retaining protrusion is squeezed.

Preferably, in the above anti-back mechanism, position retaining protrusions are arranged evenly in a circumferential direction of the push rod in units of groups.

Preferably, in the above anti-back mechanism, the number of the position retaining protrusions in each group is greater than or equal to 2. There is an offset distance between every two adjacent position retaining protrusions in each group.

Preferably, the position retaining protrusion includes a first position retaining protrusion 601a, a second position retaining protrusion 602a, a third position retaining protrusion 601b and a fourth position retaining protrusion 602b. The first position retaining protrusion 601a and the second position retaining protrusion 602a are adjacent to each other. The third position retaining protrusion 601b and the fourth position retaining protrusion 602b are symmetrically provided with respect to the push rod and provided adjacent to each other.

Preferably, in the above anti-back mechanism, a pitch between the first position retaining protrusion 601a and the second position retaining protrusion 602a ranges from 0.1mm to 1mm.

Preferably, in the above anti-back mechanism, a pitch between the third position retaining protrusion 601b and the fourth position retaining protrusion 602b ranges from 0.1mm to 1mm.

A drug delivery device is further provided according to the present application, which includes the drug delivery device according to any one of the above aspects.

Preferably, the drug delivery device further includes a cartridge holder for charging a drug and a liner provided with the push rod. The cartridge holder and the liner are connected to each other to form the drug delivery device having a cavity. An inner end of the push rod is in contact with the rubber bung in use.

Preferably, the drug delivery device further includes a pressing spring sleeved on the push rod. An end of the pressing spring is provided on the clamping groove.

The anti-back mechanism for the drug delivery device according to this solution includes the retaining groove, the position retaining protrusion and the clamping groove, the retaining groove is provided on the push rod, the position retaining protrusion is provided in the drug delivery device, and the clamping groove has an accommodation cavity into which the position retaining protrusion is inserted. The position retaining protrusion is provided on a ring having a notch and may be clamped with respect to the push rod when the position retaining protrusion is squeezed. When the position retaining protrusion is subjected to a reverse thrust from the cartridge holder of the drug delivery device, the position retaining protrusion may move in a direction opposite to an injection direction, be inserted into the clamping groove to be clamped, and then hooked to the retaining groove to be locked. In the conventional technology, when the push rod is stopped being provided with an external force, a certain thrust force may be generated by liquid and the rubber bung in the device. If the external force is no longer provided after an injection process is ended, the push rod is apt to reverse. For the anti-back mechanism according to this solution, since the cartridge holder of the drug delivery device is squeezed, the position of the position retaining protrusion is changed, the position retaining protrusion is pushed into the clamping groove and clamped to the retaining groove, finally, the position retaining protrusion is hooked to the retaining groove to be locked, thereby achieving the effects of fastening, anti-reversing and clamping. Hereby, a phenomenon of reversing of the push rod is prevented from occurring. The drug delivery device is further provided according to this solution. The anti-back mechanism can allow the push rod to remain at its original position after use. The drug delivery device employing the anti-back mechanism can prevent the push rod from reversing.

### BRIEF DESCRIPTION OF THE DRAWINGS

For more clearly illustrating embodiments of the present application or the technical solutions in the conventional technology, drawings referred to describe the embodiments or the conventional technology will be briefly described hereinafter. Apparently, the drawings in the following description are only examples of the present application, and for the person skilled in the art, other drawings may be further obtained based on the drawings without any creative efforts.
Figure 1 is a schematic view showing the structure of an anti-back mechanism according to the present application;
Figure 2 is a partially enlarged view of a position retaining protrusion in Figure 1;
Figure 3 shows an embodiment of the position retaining protrusion according to the present application;
Figure 4 shows another embodiment of the position retaining protrusion according to the present application; and
Figure 5 is a schematic view showing the structure of a drug delivery device according to the present application.

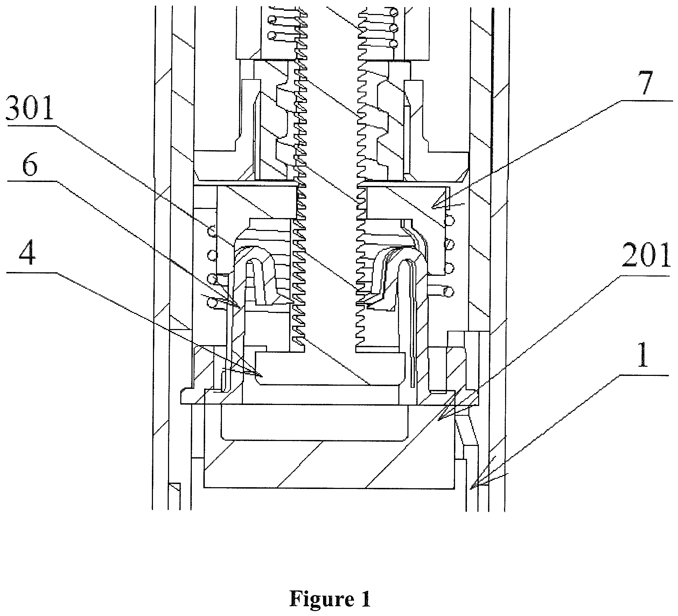

**Reference Numerals in Figures 1 to 5:**

| | | | |
|---|---|---|---|
| 1 | cartridge holder, | 2 | cartridge core, |
| 201 | rubber bung, | 301 | pressing spring, |
| 4 | push rod, | 5 | liner, |
| 6 | position retaining protrusion, | | |
| 601a | first position retaining protrusion, | | |
| 601b | second position retaining protrusion, | | |
| 602a | third position retaining protrusion, | | |
| 602b | fourth position retaining protrusion, | 7 | clamping groove. |

### DETAILED DESCRIPTION OF THE EMBODIMENTS

An aspect of the present application is to provide a drug delivery device and an anti-back mechanism thereof. The anti-back mechanism may allow a push rod to remain at its original position after use. The drug delivery device employing the anti-back mechanism may prevent the push rod from reversing.

In order to make the person skilled in the art have a better understanding of the technical solutions according to the present application, the present application is described hereinafter in further detail in conjunction with the drawings and embodiments.

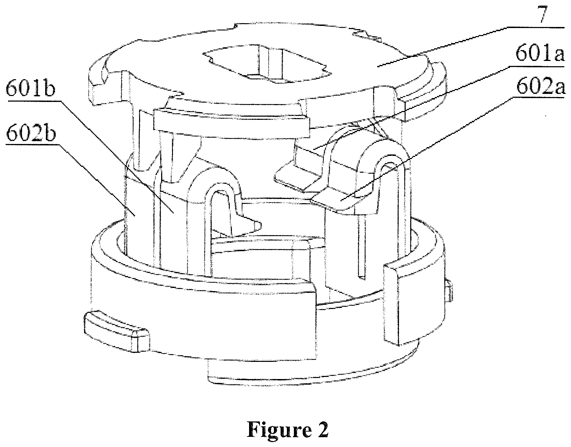

Referring to Figure 1 and Figure 2, an anti-back mechanism for a drug delivery device according to the present application includes a retaining groove, a position retaining protrusion 6 and a clamping groove 7. The retaining groove is provided on a push rod 4. The position retaining protrusion 6 is provided in the drug delivery device. The clamping groove 7 has an accommodation cavity into which the position retaining protrusion 6 may be inserted. The position retaining protrusion 6 is provided on a ring having a notch, and may be clamped with respect to the push rod 4 when the position retaining protrusion 6 is squeezed. Specifically, since the notch of the ring which is squeezed contracts inward, the position retaining protrusion 6 is brought to be clamped with respect to the push rod 4. When the position retaining protrusion 6 is subjected to a reverse thrust from a cartridge holder of the drug delivery device, the position retaining protrusion 6 may be inserted into the clamping groove 7, clamped with respect to the push rod 4, and hooked to the retaining groove on the push rod 4 to be locked. The retaining groove on the push rod is generally a groove formed by spaced protrusions provided on the push rod.

In the conventional technology, when an external force provided on the push rod 4 is stopped, a certain reverse thrust may be generated by liquid and a rubber bung 201 in the device. If the external force is no longer provided after an injection process is ended, the push rod 4 is apt to reverse. For the anti-back mechanism for the drug delivery device according to this solution, since the cartridge holder of the drug delivery device is squeezed, the position of the position retaining protrusion 6 is changed, and the position retaining protrusion 6 is pushed into the clamping groove 7 to be clamped to the retaining groove. Finally, the position retaining protrusion 6 is hooked to the retaining groove to be locked, thus achieving the effects of fastening, anti-reversing and clamping. Hereby, the push rod is prevented from reversing.

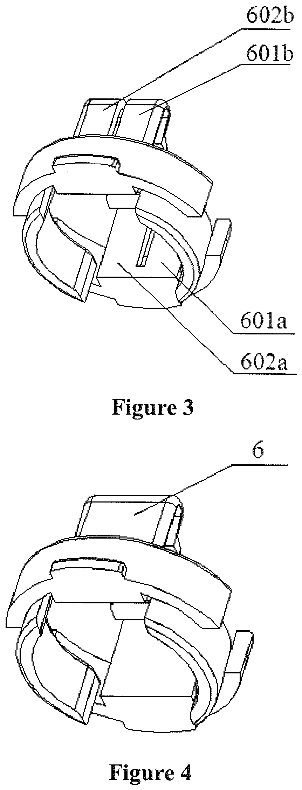

Furthermore, referring to Figure 3, position retaining protrusions 6 are evenly arranged in units of groups in a circumferential direction of the push rod 4. The number of the position retaining protrusions 6 in each group may be one, two or greater than two. As shown in Figure 4, compared with the case that a single position retaining protrusion 6 is hooked to the retaining groove to be locked, if the position retaining protrusions 6 in units of groups are evenly arranged in a manner that the positions of the position retaining protrusions 6 are retained by the retaining groove to allow the position retaining protrusions 6 to be locked, a clamping force between the position retaining protrusion and the retaining groove may be increased, and a phenomenon of reversing is more effectively prevented, while the strength of the anti-back mechanism is increased and the number of use is substantially improved.

In the case that the number of the position retaining protrusions 6 in each group is greater than or equal to 2, there is an offset distance between every two adjacent position retaining protrusions 6. The position retaining protrusions 6 in each group are spatially distributed in an axial direction of the injection pen push rod 4 at offset distances, that is, there is a pitch between every two adjacent position retaining protrusions 6 in each group in both a horizontal direction and a vertical direction.

Furthermore, referring to Figure 2 and Figure 3, in an embodiment, the position retaining protrusion 6 includes a first position retaining protrusion 601a, a second position retaining protrusion 602a, a third position retaining protrusion 601b and a fourth position retaining protrusion 602b. The third position retaining protrusion 601b and the fourth position retaining protrusion 602b are symmetrically provided with respect to the push rod 4 and provided adjacent to each other. Two groups of the position retaining protrusions 6 are employed, and the two groups of the position retaining protrusions 6 include one group of a first position retaining protrusion 601a and a second position retaining protrusion 602a as well as the other group of a third position retaining protrusion 601b and a fourth position retaining protrusion 602b. This design is simple, with this design, the position retaining protrusion 6 may be effectively locked, and a clamping force between the position retaining protrusion 6 and the push rod 4 is increased.

In the conventional technology, threaded retaining grooves of the push rod 4 are provided at too small pitches which, for example, range from 0.1mm to 1mm, due to influences of an internal space and an internal structure, in conjunction with a conventional manufacturing process and a forming process of metal and plastic materials, in operation, the push rod 4 is propelled by the external force to squeeze a rubber bung 201 of a core, and advances to push liquid inside the rubber bung 201 out. According to the principle of the reverse thrust, when the external force provided on the push rod 4 is stopped, a certain reverse thrust may be generated by the liquid in the core and the rubber bung 201. With a single retaining position, it is easy to cause the retaining groove not to be in place, and with a single retaining groove, a clamping force is not enough, resulting in inaccurate metering while resulting in the phenomenon of reversing. When a pitch, i.e. an offset distance, between the first position retaining protrusion 601a and the second position retaining protrusion 602a ranges from 0.1mm to 1mm, or a pitch, i.e. an offset distance, between the third position retaining protrusion 601b and the fourth position retaining protrusion 602b ranges from 0.1mm to 1mm, this range of the offset distance between the position retaining protrusions can allow better position retaining and locking and allow the clamping force between the position retaining protrusion and the retaining groove to be increased, thus, the phenomenon of reversing is more effectively prevented, while the strength of the anti-back mechanism is increased and the number of use is substantially increased.

A drug delivery device is further provided according to the present application. The drug delivery device includes the above anti-back mechanism, which allows the push rod to remain at its original position after use. The drug delivery device employing this anti-back mechanism may prevent the push rod from reversing.

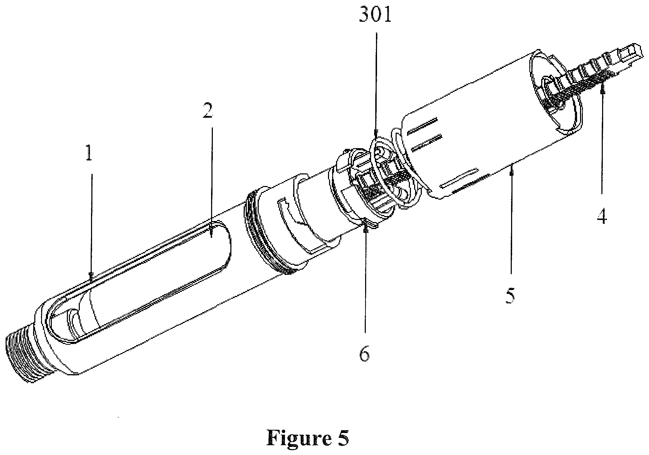

Furthermore, referring to Figure 5, the drug delivery device further includes a cartridge holder 1 for charging a drug and a liner 5 provided with the push rod 4. The cartridge holder 1 and the liner 5 are connected to each other to form the drug delivery device having a cavity. An inner end of the push rod 4 is in contact with the rubber bung 201 in use. The rubber bung 201 may push the push rod 4 to move in an axial direction of the drug delivery device. The drug delivery device may be a pen injector, a cartridge or any other container.

Still furthermore, the drug delivery device further includes a pressing spring 301 sleeved on the push rod 4. An end of the pressing spring 301 is provided on the clamping groove 7. When the pressing spring 301 is squeezed, the pressing spring 301, in a compressed state, may squeeze the position retaining protrusion 6 to allow the position retaining protrusion 6 to be inserted into the clamping groove 7, thus, the position retaining protrusion 6 is clamped with respect to the push rod 4.

It should be noted that, an injection direction described herein is a downward direction in Figure 1, and that an upward direction in Figure 1 is a direction opposite to the injection direction described herein. The definitions of the directions described above are merely for convenience of description and cannot limit the protection scope of the present application.

The drug delivery device and the anti-back mechanism thereof according to the present application are described in detail hereinbefore. Specific examples are applied herein to set forth principles and embodiments of the present application, and description of the above embodiments is only used to aid in understanding methods and core ideas of the present application. It should be noted that, for the person skilled in the art, a few of improvements and modifications can be further made to the present application without departing from principles of the present application, and these improvements and modifications also fall within the scope of claims of the present application.

## Claims

1. An anti-back mechanism for a drug delivery device, comprising:
a retaining groove provided on a push rod (4);
a position retaining protrusion (6) provided in the drug delivery device; and
a clamping groove (7) having an accommodating cavity into which the position retaining protrusion (6) is inserted,
wherein the clamping groove (7) is arranged at an end, close to a rubber bung (201), of the push rod (4), the position retaining protrusion (6) is provided on a ring having a notch, and the position retaining protrusion (6) is configured to be clamped with respect to the push rod (4) when the position retaining protrusion (6) is squeezed.

2. The anti-back mechanism according to claim 1, wherein position retaining protrusions (6) are arranged evenly in a circumferential direction of the push rod (4) in units of groups.

3. The anti-back mechanism according to claim 2, wherein the number of the position retaining protrusions (6) in each group is greater than or equal to 2, and there is an offset distance between every two adjacent position retaining protrusions (6) in each group.

4. The anti-back mechanism according to claim 3, wherein the position retaining protrusion (6) includes a first position retaining protrusion (601a), a second position retaining protrusion (602a), a third position retaining protrusion (601b) and a fourth position retaining protrusion (602b), the first position retaining protrusion (601a) and the second position retaining protrusion (602a) are adjacent to each other, and the third position retaining protrusion (601b) and the fourth position retaining protrusion (602b) are symmetrically provided with respect to the push rod (4) and provided adjacent to each other.

5. The anti-back mechanism according to claim 4, wherein a pitch between the first position retaining protrusion (601a) and the second position retaining protrusion (602a) ranges from 0.1mm to 1mm.

6. The anti-back mechanism according to claim 4, wherein a pitch between the third position retaining protrusion (601b) and the fourth position retaining protrusion (602b) ranges from 0.1 mm to 1mm.

7. A drug delivery device, comprising the anti-back mechanism according to any one of claims 1 to 6.

8. The drug delivery device according to claim 7, further comprising:
a cartridge holder (1) for charging a drug; and
a liner (5) provided with the push rod (4),
wherein the cartridge holder (1) and the liner (5) are connected to each other to form the drug delivery device having a cavity, and an inner end of the push rod (4) is in contact with the rubber bung (201) in use.

9. The drug delivery device according to claim 8, further comprising a pressing spring (301) sleeved on the push rod (4), wherein an end of the pressing spring (301) is provided on the clamping groove (7).
